# EUROPEAN PATENT SPECIFICATION

(11) **EP 2 382 069 B1**
(45) Date of publication and mention of the grant of the patent: **09.08.2017**
(21) Application number: 09764017.1
(22) Date of filing: 30.11.2009
(51) Int. Cl.: B26D 7/08, B26F 1/24, A61L 15/42, B32B 3/10

(54) **PERFORATION OF LAMINATED MATERIALS**
PERFORATION VON LAMINIERTEN MATERIALIEN
PERFORATION DE MATÉRIAUX STRATIFIÉS

(30) Priority: 28.11.2008 GB 0821702
(43) Date of publication of application: 02.11.2011
(73) Proprietor: Brightwake Limited, Nottingham, Nottinghamshire NG17 7JZ (GB)
(72) Inventor: COTTON, Stephen, Nottingham Nottinghamshire NG15 8EQ (GB)
(74) Representative: Adamson Jones
(86) International application number: PCT/GB2009/051615
(87) International publication number: WO 2010/061228

(56) References cited:
- WO-A2-2007/113597
- US-A- 5 879 494
- US-B1- 6 277 224

## Description

The present invention relates to methods of introducing perforations into a sheet of laminated material that includes a layer of hydrophobic gel. More specifically, the present invention relates to the perforation of laminates that are suitable for incorporation into wound dressings.

WO 2007/1135997 discloses releasably adhesive laminates and their use in wound dressings. Such laminates include a hydrophobic gel layer that constitutes the skin contacting layer in wound dressings. Such laminates may be perforated to permit the transmission of wound exudate through the laminate. However, because hydrophobic gels are soft and elastic, rather than rigid, it is sometimes not straightforward to introduce perforations into such a laminate in a satisfactory manner. According to the disclosure of WO 2007/1135997, the hydrophobic gel layer is perforated by either punching out small portions of the laminate, or by puncturing the laminate with pin-like perforating elements that reciprocate in and out of the gel, or are mounted on a rotating drum. Punching out portions of the laminate may potentially produce loose fragments of material that may then be unintentionally incorporated into any product that includes the perforated laminate. This is particularly undesirable when the laminate is used in the manufacture of wound dressings, where small fragments of material that become detached from a dressing could contaminate the wound. Puncturing of the laminate with perforating elements, although not leading to the production of loose fragments of material, may also be unsatisfactory, particularly for the formation of relatively small perforations, as the perforations may substantially re-occlude upon removal of the perforating element.

There has now been devised an improved method for introducing perforations into laminated sheet materials that include a layer of hydrophobic gel layer, which overcomes or substantially mitigates the above-mentioned and/or other problems associated with the prior art.

According to the invention, there is provided a method for introducing perforations into a sheet of laminated material as defined by claim 1. The application of high frequency mechanical vibrations to the sheet brings about the generation of localised heat by friction, which leads to softening of the material, thereby facilitating puncturing of the material by the perforating elements. The localised heating of the sheet, including the gel material, immediately prior to perforation, allows the gel to re-mould around the perforating element once it has punctured the material, so that once the perforating element has been withdrawn, the perforation remains substantially intact. In addition to this, the softening of the gel material reduces the force required to perforate the laminate and therefore reduces the stress that must be applied to it during perforation, reducing the risk of damage to the gel layer.

The high frequency mechanical vibrations are preferably applied to the material using a device of the type commonly used in ultrasonic welding. These devices are typically used to weld thermoplastic or fine metal components by applying high frequency mechanical vibrations to such components as they are held together under pressure. This combination of mechanical vibration and pressure results in the generation of heat by friction allowing the generation of heat to be localised to the points at which the material is held under pressure. Working materials with ultrasonics is of particular advantage in the medical industry because it does not introduce potential contaminants into the material. The use of ultrasonics is advantageous compared to the direct application of heat to the material because it is highly controllable and may be switched off instantaneously without any residual effect. Excess or residual heat is undesirable because it may damage the gel layer or cause it to deform. Also, the effects of ultrasonics can be restricted to a very limited part of the material without altering the properties of the surrounding regions. Ultrasonic techniques have previously been used for the formation of perforations in a variety of materials, including sheet materials intended for use as components of wound dressings. However, the suitability of such techniques for the formation of perforations in material comprising a hydrophobic gel is surprising, as it was to be expected that the relatively soft gel would occlude the perforations once the perforating elements are withdrawn, and that the gel would flow away from the perforating elements, resulting in irregularly-shaped perforations. Instead, it is found that well-defined and regular perforations are formed, that remain intact after their formation.

In the process of the invention, the sheet material is generally held between the perforating elements and a sonotrode, by which the high frequency vibrations are applied. The perforating elements preferably take the form of a plurality of projections extending from a support, such that the tips of the perforating elements contact the sheet material. The sonotrode may then be applied to the other side of the material so as to hold the sheet material under pressure between the sonotrode and the support, compressing the sheet material between the sonotrode surface and the projections at the points at which it is in contact with the tips of those projections. The generation of heat by friction is thereby localised to the points of the sheet material that are in contact with the tips of the perforating elements. The perforating elements may then pass through the sheet material at these points, producing perforations. The perforating elements thereby serve to compress the laminate against the sonotrode at the desired points, localising the generation of heat to the points at which they contact the laminate, followed by perforation of the laminate at those points.

The perforating elements most preferably pierce the laminate as soon as possible following contact with the sonotrode. It is therefore desirable to apply a force to the laminate to facilitate passage of the perforating elements through the laminate. This may be done by applying suction from the support, by holding the laminate under tension against the perforating elements, or by applying a mechanical force directly to the laminate.

It is preferable for the perforating elements to remain in the laminate for sufficient time to allow re-moulding of the laminate around the perforating elements. This ensures that the perforation does not re-occlude following removal of the perforating elements. Typically, the laminate remains in contact with the perforating elements for a period of between 0.1 and 5.0 seconds, more commonly between 0.1 and 1.0 seconds, or between 0.2 and 0.8 seconds, or between 0.3 and 0.6 seconds. The duration of the period for which the laminate and the perforating elements remain in contact will be a function of the form of the support and speed of throughput of the laminate.

In the process of this invention, the support from which the perforating elements extend preferably takes the form of a roller with the perforating elements extending from its circumferential surface. Such a roller will typically have a diameter of between 5cm and 50cm, more commonly between 10cm and 30cm. The laminate may be fed on and off the roller and make contact with the sonotrode continuously, improving throughput. The sonotrode must therefore apply high frequency mechanical vibrations to the material continuously. It is therefore necessary to supply the high frequency mechanical vibrations to the sonotrode using a continuous pulsating generator, rather than an intermittent pulsating generator, both of which are commonly used in the field of ultrasonics.

Generally, operation of the sonotrode for continuous periods will, unless appropriate measures are put in place to maintain the temperature of the sonotrode at a substantially constant level, result in the generation of heat and an increase in the temperature of the sonotrode. This can lead to thermal expansion of the sonotrode, which may reduce the clearance between the sonotrode and the perforating elements. It may therefore be desirable or necessary for the sonotrode to be cooled during operation, eg by the application of a cooling fluid, most commonly chilled air.

The laminate on which the process is carried out is typically in the form of an elongate strip with a width that generally does not exceed 200mm, although the use of strips with greater widths is possible. However, sonotrodes having a width of greater than about 200mm are less effective at applying high frequency mechanical vibrations to a material. Therefore, in order to perforate strips of laminate having widths in excess of 200mm, a number of sonotrodes positioned adjacent to one another may be used.

The laminate is preferably fed past the perforating elements at a rate of at least 0.1 metres/second and up to 1.0 metres/second. Typically, the laminate may be fed through the apparatus at a rate of between 0.2 and 0.8 metres/second, or between 0.3 and 0.6 metres/second.

The process of this invention is suitable for producing perforations in laminates that include any form of hydrophobic gel layer, although the gel layer is most preferably formed of a silicone gel. Silicone gels are typically formed by a reaction between two fluids that are mixed immediately prior to application to a backing layer and curing. Suitable components that are for such a reaction to form a silicone gel are freely commercially available. Typically, the two components are a vinyl substituted silicone and a hydride-containing silicone.

The thickness of the gel layer within the laminate may vary considerably but will typically be between 5µm and 10mm, but more commonly between 20µm and 5mm. The invention is particularly useful for the perforation of laminates comprising gel layers of substantial thickness, eg thicknesses of between 0.5mm and 5mm, or between 0.5mm and 2mm, eg about 1 mm or about 1.5mm.

The distribution and spacing of perforations is dependent on the distribution of perforating elements on the support. Perforations will typically be regularly arranged with a separation substantially greater than their diameter, although variation in the distribution of the perforations is possible.

The size and shape of the perforations will correspond to the size and shape of the cross section of the perforating elements. Perforations may be varied considerably in size and shape, but are typically circular and between 0.1 mm and 5mm, more commonly between 0.5mm and 2mm, in diameter, although smaller and larger perforations may be possible. However, the size of perforations may be restricted by the ability of the perforating elements to pierce the heated laminate.

Typically, perforations in any given product will all be of similar form, although it is possible for a variety of sizes and shapes of perforation to be present in a single product.

The perforations in the laminate are preferably arranged in a regular array, the perforations typically being separated by 0.2 to 10mm. Most commonly, the number of perforations per unit area is between 1 and 100, more commonly between 1 and 50, or between 1 and 20, perforations/cm². The perforations typically account for more than 5%, and up to 75%, or up to 50%, or up to 25%, of the area of the laminate.

The invention is useful in the formation of perforations in laminate materials that include a layer of hydrophobic gel, most particularly a layer of silicone gel. Such a laminate most commonly also comprises a carrier to which the gel is bonded. Usually, it will be preferred for the gel to also carry a protective sheet that, in the perforating operation, is interposed between the gel layer and the perforating elements and is therefore perforated along with the gel layer. The carrier layer will generally also be perforated, as for most applications it will be necessary for the perforations to extend through each component of the laminate that is subsequently incorporated into a composite product such as a wound dressing. Preferably, both the carrier layer and the protective sheet are sheets of synthetic thermoplastics materials.

Preferred materials for use as the carrier layer are materials with an open or irregular surface structure, into which the material of the gel layer may penetrate, thereby creating a physical bond between the carrier layer and the gel layer. Such materials include textile materials, including woven and non-woven textiles, as well as materials such as meltblown plastics. A particularly preferred material for use as the carrier layer is a meltblown polyurethane sheet. Such materials have an open structure that becomes impregnated with, and hence bonded to, the gel layer.

Preferred materials for the protective sheet are continuous films of thermoplastics. Examples of suitable thermoplastics materials include polyolefins, eg polyethylene.

Such thermoplastic materials for the carrier layer and protective sheet will melt under the influence of the sonotrode, and then solidify as the material cools after formation of the perforations.

The laminate may further comprise a substrate to facilitate transport of the laminate through the perforating apparatus. The substrate may be of a relatively inelastic material that ensures the dimensional stability of the laminate as it is fed through the perforating apparatus. The substrate may also be of greater tensile strength than the other components of the laminate, so that it maintains the integrity of the laminate during the perforation operation. Suitable materials for the substrate are papers and the like, which may be bonded to the carrier layer by means of adhesives, eg acrylic adhesive. Thus, the laminate may comprise a substrate, of paper or the like, a thermoplastic carrier layer to which is bonded the gel layer, and a protective sheet of thermoplastic material. In the perforating operation, the perforating elements contact the protective sheet, and press the substrate into contact with the sonotrode. The localised heating of the laminate material in the vicinity of the perforating elements results in the perforating elements penetrating at least the protective sheet, the gel layer and the carrier layer.

The substrate may also be perforated. However, in certain circumstances this may be disadvantageous. For instance, where the substrate is of a material such as paper, perforation of that layer results in the generation of fragments of material that may occlude the perforations or the build up of which may interfere with proper operation of the process. Thus, in preferred embodiments of the invention, the laminate is supported by a substrate that, in the perforating operation, is interposed between the carrier layer and the sonotrode, and which is not itself penetrated by the perforating elements and hence is not perforated. In such embodiments, the clearance between the perforating elements and the sonotrode is chosen such that the perforating elements penetrate the protective sheet (where present), the gel layer and the carrier layer (where present), but not the substrate. Perforation of the substrate may also be prevented by the use of a material for that layer that is not perforated under the operating conditions that lead to perforation of the other layers.

Thus, in a preferred aspect of the invention, there is provided a method for introducing perforations into a sheet of laminated as defined by claim 1. As described above, the sheet of laminated material used in this preferred embodiment of the invention may further comprise a protective sheet applied to the gel layer and/or a carrier layer interposed between the gel layer and the substrate. Both such layers will also be perforated.

The substrate may form part of finished product in which the perforated laminate is incorporated. More commonly, however, the substrate is simply a processing aid used to facilitate production of the perforated laminate, and is removed prior to incorporation of the laminate into a composite product.

Perforated hydrophobic gel layers are of particular advantage for use as skin-contacting layers in products that are in prolonged contact with the skin. Hydrophobic gels are generally impermeable to fluids, such as water vapour, resulting in discomfort and irritation when in prolonged contact with the skin. The introduction of perforations into the gel layer allows the transmission of fluids, such as water vapour, improving the breathability of the gel layer and thereby improving comfort. The perforated hydrophobic gel layer is therefore of potential utility as the skin-contact layer of a wound dressing. A wound dressing having a skin-contact layer including a perforated hydrophobic gel layer can be produced according to the process of this invention. The improved breathability of the hydrophobic gel layer allows the entire skin-contacting surface of a dressing to be coated. This provides an advantage over conventional dressings, the skin-contact layers of which are only partially coated with hydrophobic gel to allow fluid transmission, which compromises adhesion and increases the likelihood of leakage or detachment of the dressing.

It is often necessary for a dressing to be capable of transmitting wound exudate away from a wound site. Hydrophobic gel layers do not permit the free transmission of fluids. Therefore, wound dressings having a skin-contacting layer coated with hydrophobic gel generally require an opening in the gel layer to allow the transmission of wound exudate away from the wound. This invention provides a further advantage over conventional dressings by providing stronger adhesion in the regions of the dressing that are coated with gel.

The invention will now be described in greater detail, by way of illustration only, with reference to the accompanying drawings, in which:
Figure 1 is a perspective view, not to scale, of a portion of a first embodiment of a perforated laminate sheet produced according to this invention;
Figure 2 is a schematic representation, not to scale, of the apparatus used to produce the perforations in the material of Figure 1;
Figure 3 is a detailed schematic view showing the manner in which perforations are introduced into the material of Figure 1;
Figure 4 is a view similar to Figure 1 of a second embodiment of a perforated laminate sheet produced in accordance with the invention;
Figure 5 is a view similar to Figure 3 showing the manner in which perforations are introduced into the material of Figure 4;
Figure 6 is a cross-sectional view, not to scale, of a dressing that incorporates a perforated gel layer produced according to this invention; and
Figure 7 is an underside plan view of the skin-contacting surface of the dressing of Figure 6.

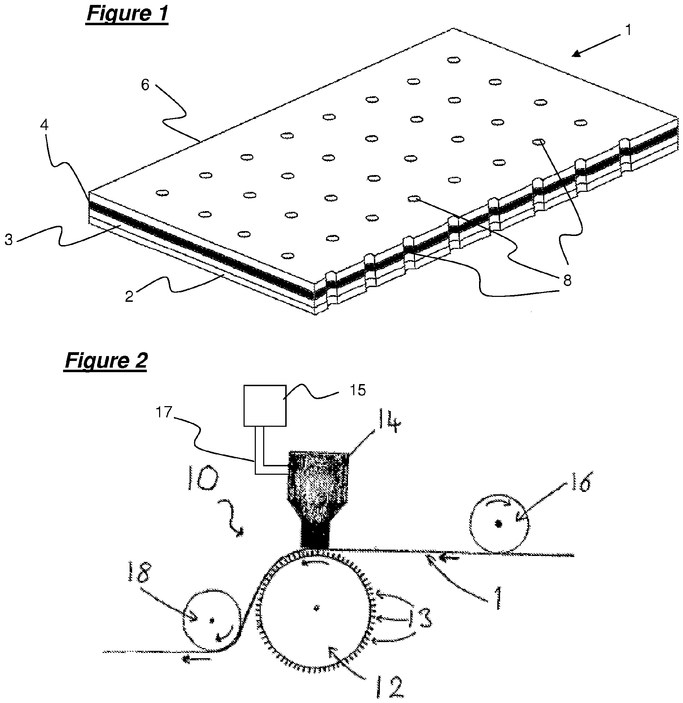

Referring first to Figure 1, a perforated laminate sheet is generally designated 1. The laminate consists of a substrate 2 that is bonded to a carrier layer 3, that in turn carries a silicone gel layer 4. The silicone gel layer 4 is covered by a protective sheet 6. The laminate 1 is completely perforated by a multitude of regularly spaced perforations 8.

The perforated laminate 1 depicted in Figure 1 may be produced as follows. First, a non-perforated laminate is manufactured. A continuous sheet of a prelaminate comprising the substrate 2 and carrier layer 3 may be fed onto a conveyor to be transported through the successive stages of the manufacturing process. The substrate 2 comprises a layer of waxed paper and the carrier layer 3 is a sheet of meltblown polyurethane. The two materials of the prelaminate are bonded by means of an acrylic adhesive. The gel layer 4 is formed by applying a curable composition to the carrier layer 3 via an applicator. Most commonly, the composition will be prepared by mixing of two components prior to application of the mixture to the carrier layer 3. Prior to curing, the mixture is fluid and can be applied to the carrier layer 3 as a uniform layer with the desired thickness. The mixture may be applied by spraying, but more commonly is applied from the edge of a suitably formed blade that is positioned close to the surface of the carrier layer 3 passing beneath it. Alternatively, the composition may simply be poured onto the carrier layer 3, which is then drawn beneath a doctor blade or bar that distributes the composition with a defined and uniform thickness.

After application of the curable silicone mixture, the coated prelaminate passes into a first curing stage where it passes beneath a bank of medium wave infra-red heaters that operate continuously. The thermal energy from these heaters initiates curing of the silicone mixture, and in particular cures the upper surface of the mixture, which maintains the structural integrity of the silicone layer during its passage through a second, longer curing stage. In the second curing stage, the coated prelaminate passes beneath further medium wave infra-red heaters. Curing of the silicone mixture, to form a gel layer 4 of the desired thickness and other properties, is completed during passage through the second curing stage. The operating parameters may be optimised to suit the particular product being manufactured. Variables that may be adjusted include the power of the infra-red heaters, the speed of passage through the various stages of the process, as well as the length of the curing stages. Typically, the passage time through the curing stages is between 5 and 15 minutes. As the silicone composition penetrates into the open structure of the meltblown polyurethane of the carrier layer 3, the cured silicone is bonded to the carrier layer 3.

Following the curing process, the protective sheet 6, which is of polyethylene, is applied to the exposed surface of the gel layer 4, forming the laminate 1 consisting of the gel layer 4 and carrier layer 3, with the substrate 2 on one side and the protective sheet 6 on the other.

Referring now to Figure 2, perforations 8 are incorporated into the laminate 1 using a perforating apparatus that is generally designated 10 and consists of a perforating roller 12 which is a cylindrical barrel having a multitude of flat-tipped, pin-like perforating elements 13 projecting from the circumferential surface, and a sonotrode 14 which, in operation, applies high frequency mechanical vibrations to the laminate 1. The perforating roller 12 and sonotrode 14 are configured so that when the perforating roller 12 is rotated, the tips of the perforating elements 13 pass close to the surface of the sonotrode 14. The diameter of the perforating roller 12 is approximately 20cm, and the perforating elements 13 have a length of approximately 5mm.

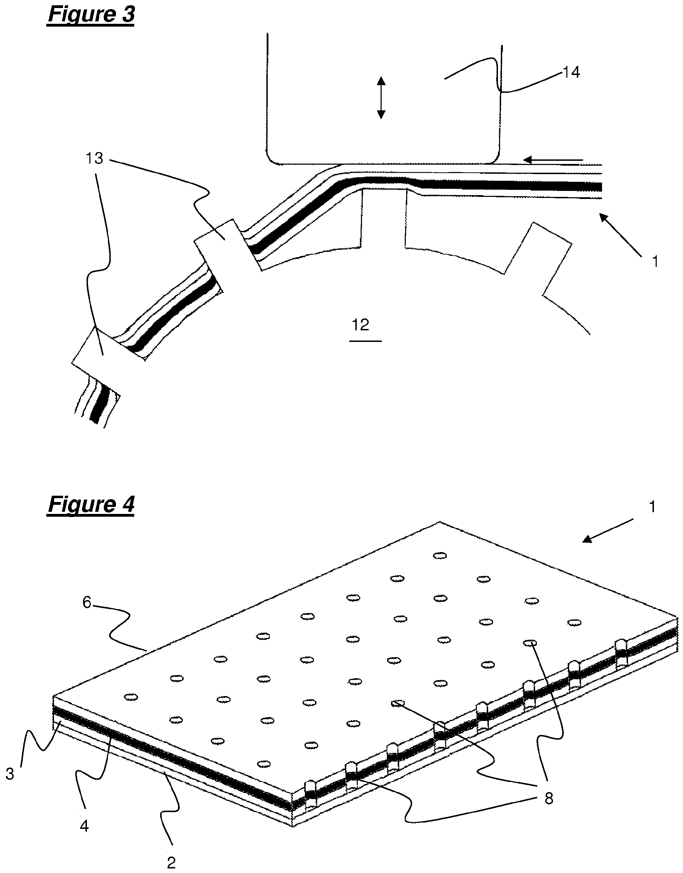

In operation, the laminate 1 is drawn (with the substrate 2 uppermost) past a guide roller 16 into the nip between the perforating roller 12 and the sonotrode 14. As is most clearly evident from Figure 3, the points at which the laminate 1 contacts the tips of the perforating elements 13 of the perforating roller 12 are compressed against the surface of the sonotrode 14. The high frequency mechanical vibrations produced by the sonotrode 14 (indicated by the doubleheaded arrow in Figure 3) generate high levels of friction at the points where laminate is compressed, causing heating of the laminate at these points. The material of the laminate 1 melts at those points were such heating occurs, allowing the perforating elements 13 to pass through the laminate 1, thereby forming the perforations 8. The protective sheet 6 on the lower surface of the laminate prevents the silicone of the gel layer 4 sticking to the roller 12 on passage through the perforating apparatus, which would potentially damage the silicone layer 4 and disrupt the perforation process.

The laminate 1 is drawn off the perforating roller 12 via a second guide roller 18. The second guide roller 18 is positioned such that the laminate 1 remains in contact with the surface of the perforating roller 12 after passing through the nip between the perforating roller 12 and the sonotrode 14. In the nip between the perforating roller 12 and the sonotrode 14, the protective sheet 6, which comes into direct contact with the penetrating elements 13, melts in the vicinity of the points of contact with the perforating elements 13 and is perforated by them, as are the gel layer 4, the carrier layer 3 and substrate 2. As the laminate 1 remains in contact with the perforating roller 12 after passing through the nip, the locally heated material of the laminate 1 cools somewhat, so that when the laminate 1 is drawn off the perforating roller 12, and hence the perforating elements 13 are withdrawn from the perforations 8 that have been formed, the integrity of the perforations 8 is maintained.

Chilled air from a chiller unit 15 is blown through the sonotrode 14 via a conduit 17. The flow of chilled air is controlled to maintain the temperature of the sonotrode 14 substantially constant, and hence prevent thermal expansion of the sonotrode 14 that would otherwise reduce the clearance between the sonotrode 14 and the tips of the perforating elements 13.

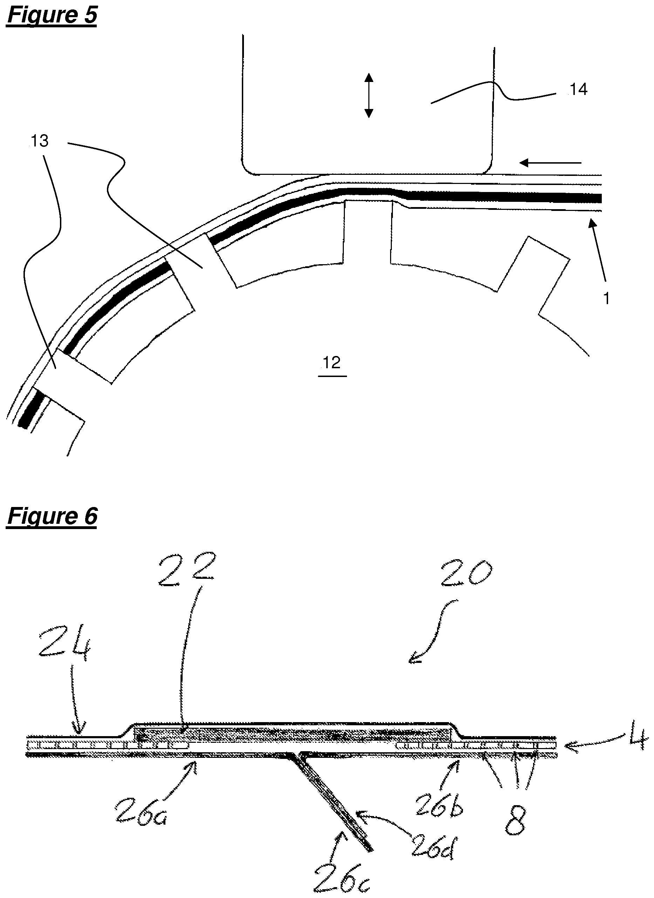

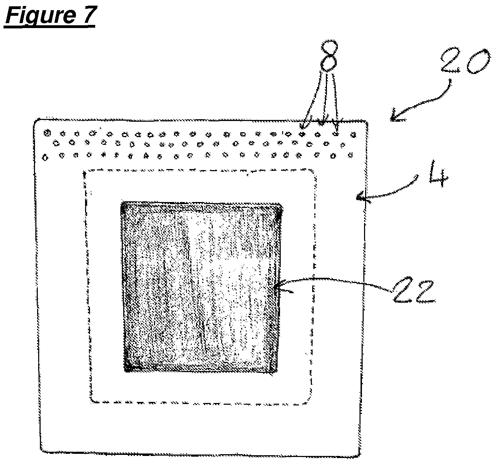

The laminate is drawn off the perforating roller 12 at a rate of approximately 0.3 metres/second. The perforated laminate 1 may be taken up on a roller (not shown) for storage or may pass directly to further processing stations for conversion to finished products such as the dressing described below in relation to Figures 6 and 7.

The position of the perforating roller 12 in relation to the sonotrode 14 may be adjusted in order to alter the size of the nip between the tips of the perforating elements and the sonotrode surface. This may be done to accommodate laminates with a variety of thicknesses, or to vary the pressure exerted on the laminate when the apparatus is in operation since an increase in pressure will generally cause an increase in friction and therefore increased generation of heat.

Figures 4 and 5 relate to another, currently preferred, embodiment of the invention. As can be seen in Figure 4, in this embodiment, the laminate 1 is of similar construction to that of Figure 1, comprising a substrate 2, a carrier layer 3, a gel layer 4 and a protective sheet 6. In this embodiment, however, the perforations 8 that are formed in the laminate extend through the protective sheet 6, the gel layer 4 and carrier layer 3, but not through the substrate 2.

As can be seen in Figure 5, the clearance between the perforating elements (pins) 13 and the sonotrode 14 is chosen such that the perforating elements 13 penetrate the the protective sheet 6, the gel layer 4 and carrier layer 3, but not the substrate 2. The benefit of this embodiment is that it does not produce fragments of the paper material used for the substrate 2.

Referring now to Figure 6, there is shown a cross-sectional view of one embodiment of a wound dressing, generally designated 20, which incorporates a perforated gel layer produced according to the invention. The dressing 20 consists of a generally square, perforated piece of meltblown polyurethane sheet (corresponding to the carrier layer 3 of the laminate described above) carrying on its underside (as viewed in Figure 6) a layer of silicone gel 4. A central region of the film is removed to form an opening 21 over which an absorbent pad 22 is positioned. This absorbent pad 22 completely covers the opening 21 and overlaps with the perimeter of the opening. The upper side of the dressing (as viewed in Figure 6) is covered with a protective permeable membrane 24, eg a polyurethane film. The gel layer 4 may have a wide range of thicknesses, depending in the specific application of the dressing into which it is incorporated. The gel layer 4 may range from as little as 5µm up to several millimetres, eg 3-4mm, in thickness. Typically, the thickness of the gel layer 4 is in the range 0.5mm to 2mm.

The underside of the dressing (as viewed in Figure 6) carries a two-part release liner 26a, 26b to prevent unwanted adhesion before use. The release liner 26a, 26b is typically formed from high density polyethylene (HDPE). The two components 26a, 26b of the release liner overlap, with a fold being formed in one of them 26a so as to create a first tab 26c that projects from the laminate, with the other 26b overlying the first tab 26c so as to form a second tab 26d. The tabs 26c, 26d can be grasped by a user to enable the components of the release liner 26a, 26b to be peeled away from the gel layer 4 prior to application of the dressing to a wound.

Figure 7 shows the skin-contacting face of the dressing of Figure 6, after the release liners 26a, 26b have been removed. The dressing is substantially square, although a variety of shapes may be used, depending on the application. The entire face, apart from the central opening 21, is constituted by the perforated gel layer 4. Only three rows of perforations 8 are represented in Figure 7, although in actual fact the entire gel layer is perforated. The absorbent pad 22 is positioned behind the gel layer 4 and covers the opening. The extent of the absorbent pad 22 on the other side of the gel layer 4 is shown by a dotted line, indicating how the absorbent pad 22 overlaps the perimeter of the opening 21. The presence of the opening 21 in the gel layer 4 allows fluid to be absorbed from the wound by the absorbent pad 22.

The dressing 20 is manufactured as follows. The starting material is a sheet of the perforated laminate 1, produced as described above, either with (as in Figure 1) or without (as in Figure 4) perforations that extend through the substrate 2. With the waxed paper substrate uppermost, a hole corresponding to the opening 21 is punched in the laminate 1. The waxed paper is then stripped away and an absorbent pad 22 placed in position over the opening 21. The pad 22 adheres to the acrylic adhesive that is exposed by removal of the waxed paper. The protective permeable membrane 24 is then applied over the entire upper surface of the dressing 20. The product is then inverted, the protective sheet 6 stripped away and replaced by the HDPE release liners 26a, 26b. Finally, the finished product is punched out of the composite sheet.

## Claims

1. A method for introducing perforations (8) into a sheet (1) of laminated material that includes a layer (4) of silicone gel, which method involves contacting perforating elements (13) with the sheet and subjecting the sheet, at least in the regions contacted with the perforating elements, to high frequency mechanical vibrations, such that the perforating elements puncture the sheet of laminated material, wherein the perforating elements remain in the sheet of laminated material for sufficient time to allow re-moulding of the silicone gel around the perforating elements.

2. A method as claimed in Claim 1, wherein the laminate remains in contact with the perforating elements for a period of between 0.1 and 5.0 seconds.

3. A method as claimed in Claim 1, wherein the laminate remains in contact with the perforating elements for a period of between 0.1 and 1.0 seconds.

4. A method as claimed in Claim 1, wherein the laminate remains in contact with the perforating elements for a period of between 0.2 and 0.8 second.

5. A method as claimed in Claim 1, wherein the laminate remains in contact with the perforating elements for a period of between 0.3 and 0.6 seconds.

6. A method as claimed in any preceding claim, wherein the sheet of laminated material is held under pressure between the perforating elements and a sonotrode (14), which applies the high frequency mechanical vibrations.

7. A method as claimed in any preceding claim, wherein the perforating elements are a plurality of projections extending from a roller (12), the perforating elements extending from the circumferential surface of the roller.

8. A method as claimed in Claim 7, wherein the sheet of laminated material remains in contact with the roller after it has been punctured by the perforating elements.

9. A method as claimed in Claim 8, wherein a guide roller causes the sheet of laminated material to remain in contact with the roller after it has been punctured by the perforating elements.

10. A method as claimed in Claim 9, wherein the thickness of the gel layer is between 5µm and 10mm, or between 20µm and 5mm.

11. A method as claimed in any preceding claim, wherein the sheet of laminated material comprises a substrate (2), a carrier layer (3), the layer (4) of silicone gel and a protective sheet (6).

12. A method as claimed in Claim 11, wherein the carrier layer (3) is a sheet of meltblown polyurethane.

13. A method as claimed in any one of Claims 11 or 12, wherein perforations are created in the gel layer but not in the substrate.

## Patentansprüche

1. Verfahren zum Einbringen von Perforationen (8) in eine Folie (1) aus laminiertem Material, das eine Schicht (4) aus Silikongel enthält, wobei das Verfahren das Inkontaktbringen der Perforierungselemente (13)
mit der Folie und das Unterziehen der Folie hochfrequenter mechanischer Schwingungen zumindest in den Bereichen, die mit den Perforierungselementen in Kontakt ist, beinhaltet, sodass die Perforierungselemente die Folie aus laminiertem Material durchstroßen, wobei die Perforierungselemente in der Folie aus laminierten Material für ausreichende Zeit bleiben, um erneutes Formen des Silikongels um die Perforierungselemente zu ermöglichen.

2. Verfahren nach Anspruch 1, wobei das Laminat mit den Perforierungselementen für eine Zeitspanne zwischen 0,1 und 5,0 Sekunden in Kontakt bleibt.

3. Verfahren nach Anspruch 1, wobei das Laminat mit den Perforierungselementen für eine Zeitspanne zwischen 0,1 und 1,0 Sekunden in Kontakt bleibt.

4. Verfahren nach Anspruch 1, wobei das Laminat mit den Perforierungselementen für eine Zeitspanne zwischen 0,2 und 0,8 Sekunden in Kontakt bleibt.

5. Verfahren nach Anspruch 1, wobei das Laminat mit den Perforierungselementen für eine Zeitspanne zwischen 0,3 und 0,6 Sekunden in Kontakt bleibt.

6. Verfahren nach einem der vorhergehenden Ansprüche, wobei die Folie aus laminiertem Material unter Druck zwischen den Perforierungselementen und einer Sonotrode (14) gehalten wird, die die hochfrequenten mechanischen Schwingungen aufbringt.

7. Verfahren nach einem der vorhergehenden Ansprüche, wobei die Perforierungselemente eine Vielzahl von Vorsprüngen sind, die sich von einer Rolle (12) erstrecken, wobei die Perforierungselemente sich von der Umfangsfläche der Rolle erstrecken.

8. Verfahren nach Anspruch 7, wobei die Folie aus laminiertem Material mit der Rolle in Kontakt bleibt, nachdem sie von den Perforierungselementen durchstoßen wurde.

9. Verfahren nach Anspruch 8, wobei eine Führungsrolle bewirkt, dass die Folie aus laminiertem Material mit der Rolle in Kontakt bleibt, nachdem sie von den Perforierungselementen durchstoßen wurde.

10. Verfahren nach Anspruch 9, wobei die Dicke der Gelschicht zwischen 5 pm und 10 mm oder zwischen 20 pm und 5 mm liegt.

11. Verfahren nach einem der vorhergehenden Ansprüche, wobei die Folie aus laminiertem Material ein Substrat (2), eine Trägerschicht (3), die Schicht (4) aus Silikongel und eine Schutzfolie (6) umfasst.

12. Verfahren nach Anspruch 11, wobei die Trägerschicht (3) eine Folie aus schmelzgeblasenem Polyurethan ist.

13. Verfahren nach einem der Ansprüche 11 oder 12, wobei Perforationen in der Gelschicht, aber nicht in dem Substrat erzeugt werden.

## Revendications

1. Procédé pour pratiquer des perforations (8) dans une feuille (1) de matériau stratifié qui comprend une couche (4) de gel de silicone, ledit procédé consistant à placer des éléments de perforation (13) en contact avec la feuille et à soumettre la feuille, au moins dans les régions en contact avec les éléments de perforation, à des vibrations mécaniques à haute fréquence, de sorte que les éléments de perforation perforent la feuille de matériau stratifié, lesdits éléments de perforation restant dans la feuille de matériau stratifié pendant un temps suffisant afin de permettre au gel de silicone de se mouler de nouveau autour des éléments de perforation.

2. Procédé selon la revendication 1, ledit stratifié restant en contact avec les éléments de perforation pour une durée comprise entre 0,1 et 5,0 secondes.

3. Procédé selon la revendication 1, ledit stratifié restant en contact avec les éléments de perforation pour une durée comprise entre 0,1 et 1,0 seconde.

4. Procédé selon la revendication 1, ledit stratifié restant en contact avec les éléments de perforation pour une durée comprise entre 0,2 et 0,8 seconde.

5. Procédé selon la revendication 1, ledit stratifié restant en contact avec les éléments de perforation pour une durée comprise entre 0,3 et 0,6 seconde.

6. Procédé selon l'une quelconque des revendications précédentes, ladite feuille de matériau stratifié étant maintenue sous pression entre les éléments de perforation et une sonotrode (14) appliquant les vibrations mécaniques à haute fréquence.

7. Procédé selon l'une quelconque des revendications précédentes, lesdits éléments de perforation étant une pluralité de projections s'étendant à partir d'un rouleau (12), lesdits éléments de perforation s'étendant à partir de la surface circonférentielle du rouleau.

8. Procédé selon la revendication 7, ladite feuille de matériau stratifié restant en contact avec le rouleau après avoir été perforée par les éléments de perforation.

9. Procédé selon la revendication 8, un rouleau de guidage amenant la feuille de matériau stratifié à rester en contact avec le rouleau après que celle-ci a été perforée par les éléments de perforation.

10. Procédé selon la revendication 9, l'épaisseur de la couche de gel étant comprise entre 5 µm et 10 mm, ou entre 20 µm et 5 mm.

11. Procédé selon l'une quelconque des revendications précédentes, ladite feuille de matériau stratifié comprenant un substrat (2), une couche de support (3), la couche (4) de gel de silicone et une feuille de protection (6).

12. Procédé selon la revendication 11, ladite couche de support (3) étant une feuille de polyuréthane obtenue par fusion-soufflage.

13. Procédé selon la revendication 11 ou 12, lesdites perforations étant créées dans la couche de gel mas pas dans le substrat.
